(19) Europäisches Patentamt
European Patent Office
Office européen des brevets

(11) Veröffentlichungsnummer: **0 200 146**
**A2**

(12) # EUROPÄISCHE PATENTANMELDUNG

(21) Anmeldenummer: 86105570.5

(22) Anmeldetag: 22.04.86

(51) Int. Cl.[4]: C 07 D 249/08
A 01 N 53/00
//A01N43/653

(30) Priorität: 03.05.85 DE 3515868

(43) Veröffentlichungstag der Anmeldung:
05.11.86 Patentblatt 86/45

(84) Benannte Vertragsstaaten:
AT BE CH DE FR GB IT LI NL

(71) Anmelder: BAYER AG
Konzernverwaltung RP Patentabteilung
D-5090 Leverkusen 1 Bayerwerk(DE)

(72) Erfinder: Priesnitz, Uwe, Dr.
Severinstrasse 58
D-5650 Solingen 1(DE)

(72) Erfinder: Brandes, Wilhelm, Dr.
Eichendorffstrasse 3
D-5653 Leichlingen(DE)

(54) **Permethrinsäure-(triazolyl-alkyl)-ester.**

(57) Neue Permethrinsäure-(triazolyl-alkyl)-ester der Formel

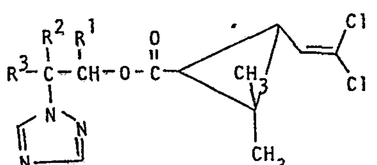

(I)

in welcher

$R^1$ für gegebenenfalls durch Halogen substituiertes tert.-Butyl steht,

$R^2$ für Wasserstoff steht und

$R^3$ für Alkyl, Alkoxy, Cycloalkylalkyl, gegebenenfalls substituiertes Aryl, gegebenenfalls substituiertes Aralkyl, gegebenenfalls substituiertes Aroxy oder gegebenenfalls substituiertes Aroxyalkyl steht oder

$R^2$ und $R^3$ gemeinsam für die Gruppe der Formel

$R^4$
$=C$ stehen, worin
$R^5$

$R^4$ für Wasserstoff, Alkyl, Cycloalkyl oder Cycloalkenyl steht und

$R^5$ für Alkyl, Cycloalkyl, Cycloalkenyl oder gegebenenfalls substituiertes Aryl steht oder

$R^4$ und $R^5$ gemeinsam mit dem Kohlenstoffatom, an das sie gebunden sind, für Cycloalkyl oder Cycloalkenyl stehen,

ein Verfahren zur Herstellung der neuen Stoffe und deren Verwendung als Fungizide.

EP 0 200 146 A2

Croydon Printing Company Ltd.

BAYER AKTIENGESELLSCHAFT 5090 Leverkusen, Bayerwerk
Konzernverwaltung RP
Patentabteilung Dü/by-c
Ia

## Permethrinsäure-(triazolyl-alkyl)-ester

Die Erfindung betrifft neue Permethrinsäure-(triazolyl-alkyl)-ester, ein Verfahren zu deren Herstellung und deren Verwendung als Fungizide.

Es ist bereits bekannt geworden, daß zahlreiche Azol-Derivate, die eine Acyl-Gruppe in β-Stellung zum Azolyl-Rest enthalten, zur Bekämpfung von Pilzen geeignet sind (vgl. EP-OS 0 015 387). Die Wirksamkeit dieser Stoffe ist jedoch bei geringen Aufwandmengen nicht immer befriedigend.

Es wurden nun neue Permethrinsäure-(triazolyl-alkyl)-ester der Formel

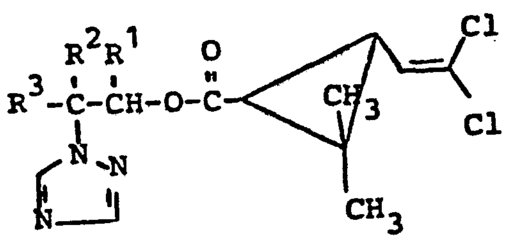

(I)

in welcher

$R^1$ für gegebenenfalls durch Halogen substituiertes tert.-Butyl steht,

$R^2$ für Wasserstoff steht und

$R^3$ für Alkyl, Alkoxy, Cycloalkylalkyl, gegebenenfalls substituiertes Aryl, gegebenenfalls substituiertes Aralkyl, gegebenenfalls substituiertes Aroxy oder gegebenenfalls substituiertes Aroxyalkyl steht oder

$R^2$ und $R^3$ gemeinsam für die Gruppe der Formel

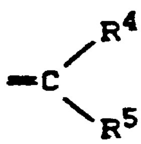

stehen, worin

$R^4$ für Wasserstoff, Alkyl, Cycloalkyl oder Cycloalkenyl steht und

$R^5$ für Alkyl, Cycloalkyl, Cycloalkenyl oder gegebenenfalls substituiertes Aryl steht oder

$R^4$ und $R^5$ gemeinsam mit dem Kohlenstoffatom, an das sie gebunden sind, für Cycloalkyl oder Cycloalkenyl stehen,

gefunden.

Die erfindungsgemäßen Permethrinsäure-(triazolyl-alkyl)-ester der Formel (I) enthalten zumindest drei

asymmetrisch substituierte Kohlenstoffatome. Sie können daher in Form von Racematen, Diastereomeren-Gemischen oder auch in Form von optisch aktiven Enantiomeren vorliegen. Die Erfindung betrifft sowohl die Racemate als auch Diastereomeren-Gemische und optisch aktive Enantiomere.

Weiterhin wurde gefunden, daß man Permethrinsäure-(triazolyl-alkyl)-ester der Formel (I) erhält, wenn man Azolderivate der Formel

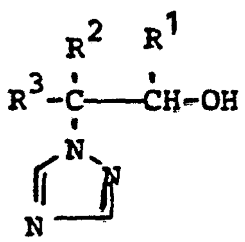

(II)

in welcher

$R^1$, $R^2$ und $R^3$ die oben angegebene Bedeutung haben,

mit Permethrinsäurehalogeniden der Formel

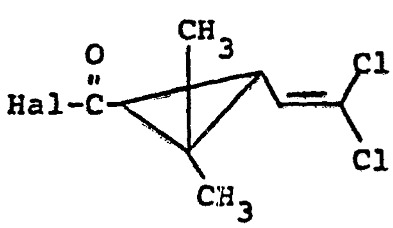

(III)

in welcher

Hal für Chlor oder Brom steht,

gegebenenfalls in Gegenwart eines Säurebindemittels und gegebenenfalls in Gegenwart eines Verdünnungsmittels umsetzt.

Schließlich wurde gefunden, daß die erfindungsgemäßen Permethrinsäure-(triazolyl-alkyl)-ester der Formel (I) sehr gute fungizide Eigenschaften besitzen.

Es ist als äußerst überraschend zu bezeichnen, daß die erfindungsgemäßen Permethrinsäure-(triazolyl-alkyl)-ester der Formel (I) eine deutlich bessere fungizide Wirksamkeit aufweisen als die konstitutionell ähnlichsten, aus dem Stand der Technik vorbekannten Stoffe gleicher Wirkungsrichtung.

Die erfindungsgemäßen Permethrinsäure-(triazolyl-alkyl)-ester sind durch die Formel (I) allgemein definiert. In dieser Formel steht $R^1$ vorzugsweise für die Gruppierung

$CH_2X^1$
|
$-C-CH_3$ , in welcher
|
$CH_2X^2$

$X^1$ und $X^2$ gleich oder verschieden sein können und für Wasserstoff, Fluor, Chlor oder Brom stehen. $R^2$ steht für Wasserstoff und $R^3$ steht vorzugsweise für Alkyl mit 1 bis 6 Kohlenstoffatomen, Alkoxy mit 1 bis 6 Kohlenstoffatomen, Cycloalkylalkyl mit 3 bis 8 Kohlenstoffatomen in der Cycloalkylgruppe und 1 bis 4 Kohlenstoffatomen im Alkylteil, gegebenenfalls ein- oder mehrfach, gleich-

stoffatomen, Halogen, Phenyl und/oder Nitro substituiertes Aryl mit 6 bis 10 Kohlenstoffatomen, gegebenenfalls ein- oder mehrfach, gleichartig oder verschieden durch Alkyl mit 1 bis 4 Kohlenstoffatomen, Halogen, Phenyl und/oder Nitro substituiertes Aralkyl mit 6 bis 10 Kohlenstoffatomen im Arylteil und 1 bis 4 Kohlenstoffatomen im Alkylteil, für gegebenenfalls ein- oder mehrfach, gleichartig oder verschieden durch Alkyl mit 1 bis 4 Kohlenstoffatomen, Halogen, Phenyl und/oder Nitro substituiertes Aroxy mit 6 bis 10 Kohlenstoffatomen oder für gegebenenfalls ein- oder mehrfach, gleichartig oder verschieden durch Alkyl mit 1 bis 4 Kohlenstoffatomen, Halogen, Phenyl und/oder Nitro substituiertes Aroxyalkyl mit 6 bis 10 Kohlenstoffatomen im Aroxyteil und 1 bis 4 Kohlenstoffatomen im Alkylteil. Außerdem stehen $R^2$ und $R^3$ auch gemeinsam für die Gruppe

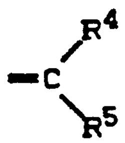 , in welcher

$R^4$ vorzugsweise für Wasserstoff, Alkyl mit 1 bis 4 Kohlenstoffatomen, Cycloalkyl mit 3 bis 8 Kohlenstoffatomen oder Cycloalkenyl mit 5 bis 8 Kohlenstoffatomen steht und $R^5$ vorzugsweise für Alkyl mit 1 bis 4 Kohlenstoffatomen, Cycloalkyl mit 3 bis 8 Kohlenstoffatomen, Cycloalkenyl mit 5 bis 8 Kohlenstoffatomen oder für gegebenenfalls ein- oder mehrfach, gleichartig oder verschieden durch Alkyl mit 1 bis 4 Kohlenstoffatomen und/oder Halogen substituiertes Aryl mit 6 bis 10 Kohlenstoffatomen steht, und außerdem $R^4$ und $R^5$ auch gemeinsam mit dem Kohlenstoffatom, an das sie gebunden sind, vorzugsweise für Cycloalkyl mit 5 bis 8 Kohlenstoffatomen oder für Cycloalkenyl mit 5 bis 8 Kohlenstoffatomen stehen.

Besonders bevorzugt sind Stoffe der Formel (I), in denen $R^1$ für die Gruppierung

$CH_2X^1$
|
$-C-CH_3$ steht, worin
|
$CH_2X^2$

$X^1$ für Wasserstoff, Fluor oder Chlor steht und

$X^2$ für Wasserstoff, Fluor oder Chlor steht,

$R^2$ für Wasserstoff steht und

$R^3$ für Alkyl mit 1 bis 4 Kohlenstoffatomen, Alkoxy mit 1 bis 4 Kohlenstoffatomen, Cycloalkylalkyl mit 5 bis 7 Kohlenstoffatomen im Cycloalkylteil und 1 bis 3 Kohlenstoffatomen im Alkylteil, gegebenenfalls ein- bis dreifach, gleichartig oder verschieden durch Methyl, Ethyl, Propyl, Fluor, Chlor, Brom, Phenyl und/oder Nitro substituiertes Phenyl, für gegebenenfalls ein- bis dreifach, gleichartig oder verschieden durch Methyl, Ethyl, Propyl, Fluor, Chlor, Brom, Phenyl und/oder Nitro substituiertes Phenylalkyl mit 1 oder 2 Kohlenstoffatomen im Alkylteil, für gegebenenfalls ein- bis dreifach, gleichartig oder verschieden durch Methyl, Ethyl, Propyl, Fluor, Chlor, Brom, Phenyl und/oder Nitro substituiertes Phenoxy, oder für gegebenenfalls

ein- bis dreifach, gleichartig oder verschieden durch Methyl, Ethyl, Propyl, Fluor, Chlor, Brom, Phenyl und/oder Nitro substituiertes Phenoxyalkyl mit 1 oder 2 Kohlenstoffatomen im Alkylteil steht oder

$R^2$ und $R^3$ gemeinsam für die Gruppe

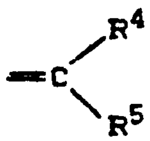

stehen, in welcher

$R^4$ für Wasserstoff, Methyl, Ethyl, Propyl, Cyclopentyl, Cyclohexyl, Cyclopentenyl oder Cyclohexenyl steht und

$R^5$ für Methyl, Ethyl, Propyl, Cyclopentyl, Cyclohexyl, Cyclopentenyl, Cyclohexenyl oder für gegebenenfalls ein- bis dreifach, gleichartig oder verschieden durch Methyl, Ethyl, Propyl, Fluor und/oder Chlor substituiertes Phenyl steht und außerdem

$R^4$ und $R^5$ auch gemeinsam mit dem Kohlenstoffatom, an das sie gebunden sind, für Cyclopentyl, Cyclohexyl, Cyclopentenyl oder Cyclohexenyl stehen.

Eine besonders bevorzugte Gruppe erfindungsgemäßer Verbindungen sind diejenigen Permethrinsäure-(triazolylalkyl)-ester der Formel

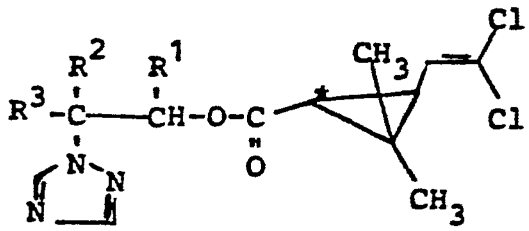

(Ia)

in welcher

$R^1$, $R^2$ und $R^3$ die oben vorzugsweise angegebene Bedeutung haben und das durch ein (*) gekennzeichnete Kohlenstoffatom im Permethrinsäure-Teil die R-Konfiguration aufweist.

Diese Stoffe können in Form von Diastereomeren-Gemischen oder auch in Form einzelner optisch aktiver Enantiomerer vorliegen.

Eine weitere Gruppe besonders bevorzugter erfindungsgemäßer Verbindungen sind diejenigen Permethrinsäure-(triazolyl-alkyl)-ester der Formel

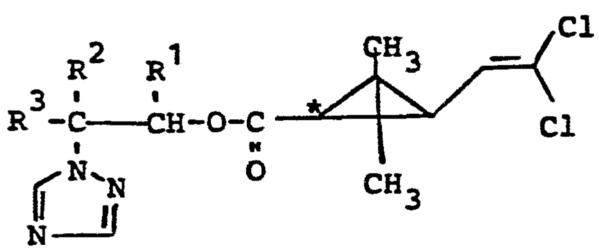

(Ib)

in welcher

$R^1$,$R^2$ und $R^3$ die oben vorzugsweise angegebene Bedeutung haben und das durch ein (*) gekennzeichnete Kohlenstoffatom im Permethrinsäure-Teil die S-Konfiguration aufweist. Diese Stoffe können in Form von Diastereomeren-Gemischen oder auch in Form einzelner optisch aktiver Enantiomerer vorliegen.

Verwendet man 1-(4-Chlor-phenyl)-2-(1,2,4-triazol-1-yl)-3-hydroxy-4,4-dimethyl-pentan und racemisches trans-Permethrinsäurechlorid als Ausgangsstoffe, so kann der Verlauf des erfindungsgemäßen Verfahrens durch das folgende Formelschema wiedergegeben werden.

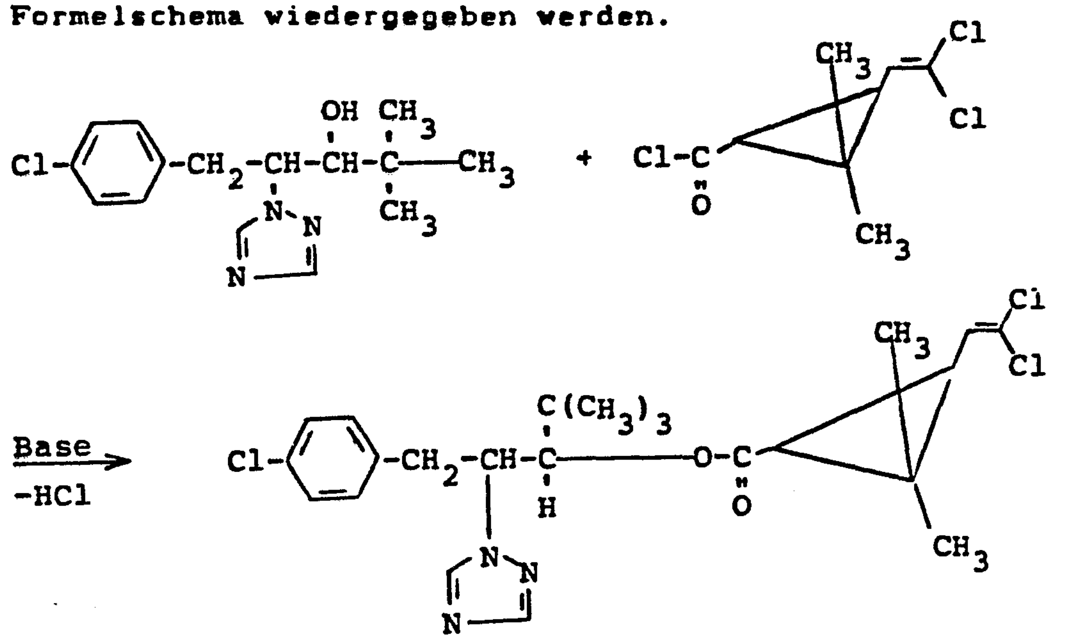

Verwendet man 1-(4-Chlor-phenoxy)-1-(1,2,4-triazol-1-yl)-2-hydroxy-3,3-dimethyl-butan und 1-R-trans-Permethrinsäurechlorid als Ausgangsstoffe, so kann der Verlauf des erfindungsgemäßen Verfahrens durch das folgende Formelschema wiedergegeben werden.

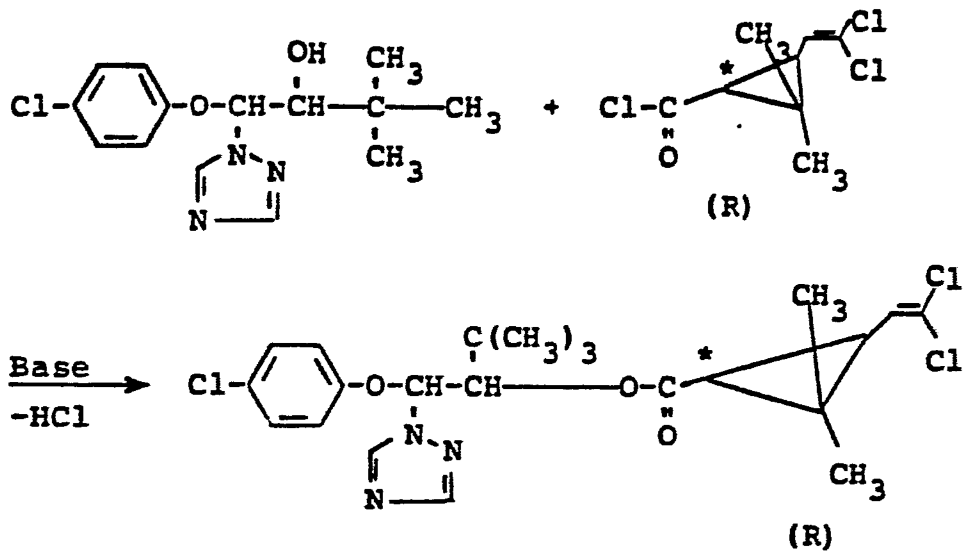


Verwendet man 1-Cyclohexyl-2-(1,2,4-triazol-1-yl)-3-hydroxy-4,4-dimethyl-pentan und 1-S-trans-Permethrinsäurechlorid als Ausgangsstoffe, so kann der Verlauf des erfindungsgemäßen Verfahrens durch das folgende Formelschema wiedergegeben werden.

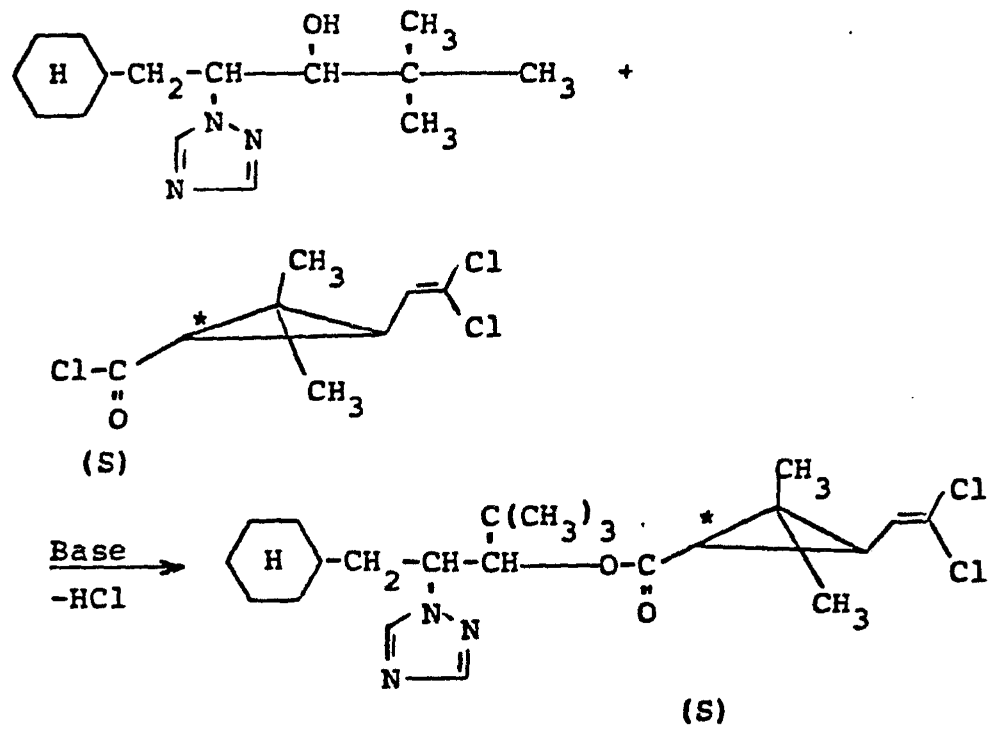


Die bei der Durchführung des erfindungsgemäßen Verfahrens als Ausgangsstoffe benötigten Azolderivate sind durch die Formel (II) definiert. In dieser Formel haben $R^1$, $R^2$ und $R^3$ vorzugsweise diejenigen Bedeutungen, die bereits im Zusammenhang mit der Beschreibung der erfindungsgemäßen Stoffe der Formel (I) vorzugsweise für diese Reste genannt wurden.

Die Azolderivate der Formel (II) sind bereits bekannt (vgl. DE-OS 2 737 489, EP-OS 0 015 387, EP-OS 0 032 200, EP-OS 0 044 425 und EP-OS 0 053 311).

Die bei der Durchführung des erfindungsgemäßen Verfahrens weiterhin als Ausgangsstoffe benötigten Permethrinsäurehalogenide sind durch die Formel (III)

definiert. Besonders bevorzugt sind Permethrinsäurechloride. Beispielhaft genannt seien racemisches trans-Permethrinsäurechlorid, 1-R-trans-Permethrinsäurechlorid und 1-S-trans-Permethrinsäurechlorid.

Die Permethrinsäurehalogenide der Formel (III) sind bereits bekannt (vgl. EP-OS 0 022 972).

Als Säurebindemittel kommen bei der Durchführung des erfindungsgemäßen Verfahrens alle üblichen anorganischen und organischen Säureakzeptoren in Betracht. Vorzugsweise verwendbar sind Alkalicarbonate, wie beispielsweise Natriumcarbonat oder Natriumhydrogencarbonat, ferner niedere tertiäre Alkylamine, Cycloalkylamine, Arylalkylamine oder Arylamine, wie beispielsweise Triethylamin, N,N-Dimethylbenzylamin, Pyridin, 1,4-Diazabicyclo-[2,2,2]- octan oder 1,5-Diazabicyclo-[4,3,0]-non-5-en.

Als Verdünnungsmittel können bei der Durchführung des erfindungsgemäßen Verfahrens alle üblichen inerten organischen Solventien eingesetzt werden. Vorzugsweise in Frage kommen Kohlenwasserstoffe, wie Benzin, Benzol, Toluol oder Xylol, halogenierte Kohlenwasserstoffe, wie Dichlormethan, Chloroform, Tetrachlorkohlenstoff, Chlorbenzol oder Dichlorbenzol, Ether wie Diethylether, Tetrahydrofuran oder Dioxan, Nitrile, wie Acetonitril oder Propionitril, oder Ester, wie Essigsäureethylester.

Die Reaktionstemperaturen können bei der Durchführung des erfindungsgemäßen Verfahrens in einem größeren Bereich variiert werden. Im allgemeinen arbeitet man bei Temperaturen zwischen -10°C und +150°C, vorzugsweise zwischen 0°C und +100°C.

Das erfindungsgemäße Verfahren wird im allgemeinen unter Normaldruck durchgeführt. Es ist jedoch auch möglich, bei erhöhtem oder vermindertem Druck zu arbeiten.

Bei der Durchführung des erfindungsgemäßen Verfahrens setzt man auf 1 Mol an Azolderivat der Formel (II) im allgemeinen 1 bis 2 Mol, vorzugsweise 1 bis 1,5 Mol, an Permethrinsäurehalogenid der Formel (III) sowie gegebenenfalls 1,5 bis 2,5, vorzugsweise 1,5 bis 2 Mol an Säurebindemittel ein. Die Aufarbeitung erfolgt nach üblichen Methoden.

Setzt man bei dem erfindungsgemäßen Verfahren ein racemisches Azolderivat der Formel (II) mit einem optisch aktiven Permethrinsäurehalogenid der Formel (III) um, so erhält man ein Diastereomeren-Gemisch von Permethrinsäure-(triazolyl-alkyl)-estern der Formel (I). So liefert z.B. die Umsetzung von racemischem erythro/threo-Gemisch von 1-(4-Chlorphenyl)-1-(1,2,4-triazol-1-yl)-2-hydroxy-3,3-dimethyl- butan mit 1-R-trans--Permethrinsäurechlorid ein Diastereomeren-Gemisch von Estern. Der Ablauf dieser Umsetzung kann durch das folgende Formelschema veranschaulicht werden:

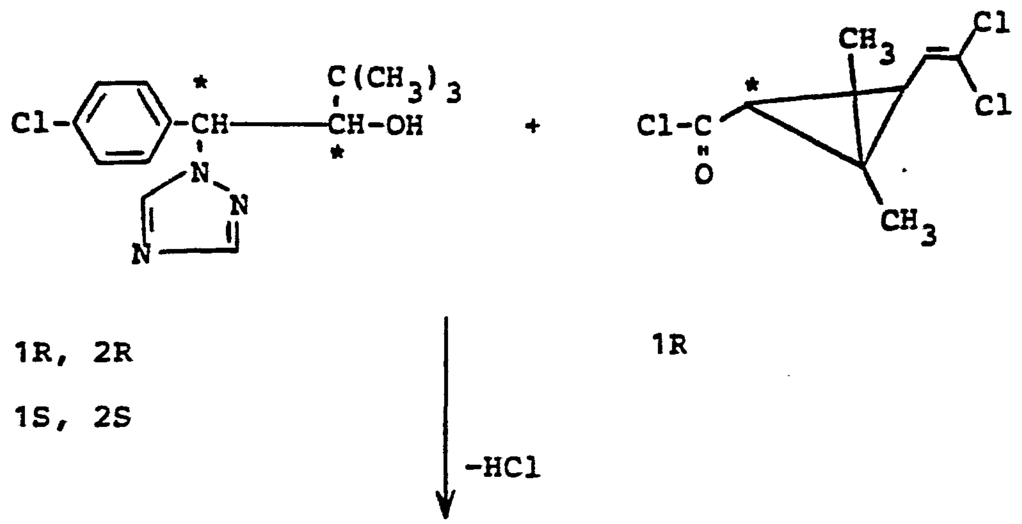


1R,2R-1R-trans-Ester + 1S,2S-1R-trans-Ester

Durch Umsetzung mit 1-S-trans-Permethrinsäurechlorid entsteht ein Diastereomeren-Gemisch der entsprechenden 1-S-trans-Ester.

Die bei dem erfindungsgemäßen Verfahren anfallenden Diastereomeren-Gemische lassen sich nach üblichen Methoden trennen, also z.B. durch fraktionierte Kristallisation oder auch mit Hilfe von chromatographischen Verfahren.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen und bakteriellen Erkrankungen, die unter die oben aufgezählten Oberbegriffe fallen, genannt.

Xanthomonas-Arten, wie Xanthomonas oryzae;
Pseudomonas-Arten, wie Pseudomonas lachrymans;
Erwinia-Arten, wie Erwinia amylovora;
Pythium-Arten, wie Pythium ultimum;
Phytophthora-Arten, wie Phytophthora infestans;
Pseudoperonospora-Arten, wie Pseudoperonospora humuli oder Pseudoperonospora cubense;
Plasmopara-Arten, wie Plasmopara viticola;
Peronospora-Arten, wie Peronospora pisi oder P. brassicae;
Erysiphe-Arten, wie Erysiphe graminis;
Sphaerotheca-Arten, wie Sphaerotheca fuliginea;
Podosphaera-Arten, wie Podosphaera leucotricha;
Venturia-Arten, wie Venturia inaequalis;
Pyrenophora-Arten, wie Pyrenophora teres oder P. graminea;
(Konidienform: Drechslera, Syn: Helminthosporium);
Cochliobolus-Arten, wie Cochliobolus sativus;
(Konidienform: Drechslera, Syn: Helminthosporium);
Uromyces-Arten, wie Uromyces appendiculatus;
Puccinia-Arten, wie Puccinia recondita;
Tilletia-Arten, wie Tilletia caries;

Ustilago-Arten, wie Ustilago nuda oder Ustilago avenae; Pellicularia-Arten, wie Pellicularia sasakii; Pyricularia-Arten, wie Pyricularia oryzae; Fusarium-Arten, wie Fusarium culmorum; Botrytis-Arten, wie Botrytis cinerea; Septoria-Arten, wie Septoria nodorum; Leptosphaeria-Arten, wie Leptosphaeria nodorum; Cercospora-Arten, wie Cercospora canescens; Alternaria-Arten, wie Alternaria brassicae; Pseudocercosporella-Arten, wie Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur Bekämpfung von Gemüsekrankheiten, wie beispielsweise den Erreger Sphaerotheca fuliginea, einsetzen.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkanpselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten, oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene

und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder den daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei der Behandlung des Bodens sind Wirkstoffkonzentrationen von 0,00001 bis 0,1 Gew.-%, vorzugsweise von 0,0001 bis 0,02 Gew.-%, am Wirkungsort erforderlich.

Die Herstellung und die Verwendung der erfindungsgemäßen Wirkstoffe geht aus den folgenden Beispielen hervor.

## Herstellungsbeispiele

### Beispiel 1

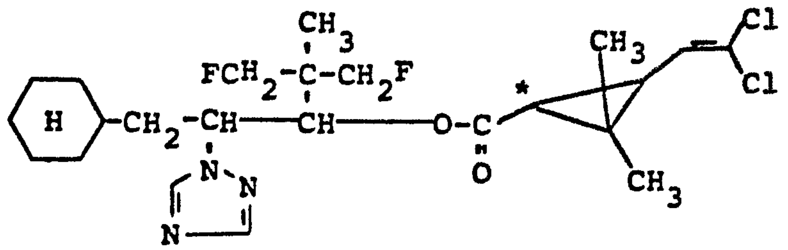

(I-1)

1R trans

Ein Gemisch von 760 mg (3,3 mMol) 1R-trans-Permethrinsäurechlorid und 1 g (3,3 mMol) 1-Cyclohexyl-2-(1,2,4-triazol-1-yl)-3-hydroxy-4,4-bis-fluormethylpentan wird 12 Stunden bei 100°C gerührt. Danach wird mit 5 ml Triethylamin versetzt. Zu dem resultierenden Reaktionsgemisch werden Diethylether und Wasser hinzugefügt. Die organische Phase wird abgetrennt, getrocknet und unter vermindertem Druck eingedampft. Man erhält auf diese Weise 1,5 g eines honiggelben Produktes, das gemäß Gaschromatogramm zu 92 % aus einem Diastereomeren-Gemisch von Verbindungen der oben angegebenen Formel (I-1) besteht. Danach errechnet sich die Ausbeute zu 87 % der Theorie.

$H^1$-Kernresonanzspektrum (90 MHz):
δ = 0,57 (3H, sbr); δ = 0,8 - 1,8 (20H,mbr.)
δ = 2,2 (1H, 2 d.d.); δ = 3,8 - 4,8 (5H,mbr.)
δ = 5,2 (1H, d.); δ = 5,7 und 5,8 (1H, 2d)
δ = 7,9 (1H,s); δ = 8,14 und 8,20 (1H,2s).

Beispiel 2

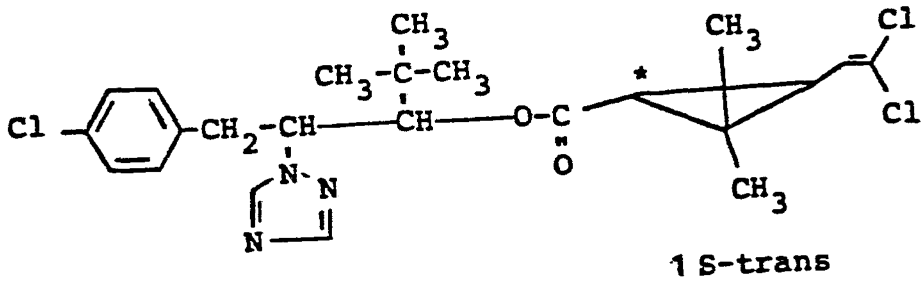

(I-2)

1 S-trans

Ein Gemisch aus 1 g (4,4 mMol) 1R-trans-Permethrinsäurechlorid, 1,25 g (4,2 mMol) 1-(4-Chlor-phenyl)-2-(1,2,4-triazol-1-yl)-3-hydroxy-4,4-dimethyl-pentan und 10 ml Pyridin wird 2 Stunden bei 110 bis 130°C gerührt. Danach

wird mit Toluol und Wasser versetzt. Die organische Phase wird abgetrennt, getrocknet und unter vermindertem Druck eingedampft. Man erhält auf diese Weise 2,1 g eines honiggelben, kristallinen Produktes, das gemäß gaschromatographischer Analyse zu 80 % aus einem Diastereomeren-Gemisch von Estern der Formel (I-2) besteht. Demnach errechnet sich die Ausbeute zu 84 % der Theorie.

$H^1$-NMR-Spektrum (90 MHz):
δ = 0,74 und 0,80 (9H,2S);
δ = 1,24, 1,30, 1,32 und 1,39 (6H,4s);
δ = 1,75 und 1,77 (1H,2d);
δ = 2,36 (1H,2 dd); δ = 3,0 (2H,2d);
δ = 4,72 - 4,95 (1H,m); δ = 5,09 (1H,2d);
δ = 5,71 und 5,74 (1H,2d; δ = 6,8 bis 7,2 (4H,d);
δ = 7,8(1H,s); δ = 8,13 und 8,19 (1H,2s).

Nach den zuvor angegebenen Methoden werden auch die in den folgenden Beispielen aufgeführten Stoffe hergestellt.

<u>Beispiel 3</u>

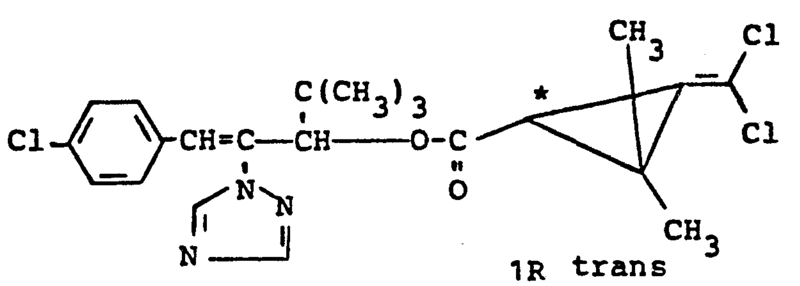

1R trans

(I-3)

Diastereomeren-Gemisch

$H^1$-NMR-Spektrum (60 MHz):

δ = 0,7 (9H,2s); δ = 1,3 (6H,2s)
δ = 1,73 (1H,2d); δ = 2,35 (1H,2dd)
δ = 5,62 und 5,76 (1H,2s)
δ = 5,98 (1H,d); δ = 7,16 - 7,48 (5H)
δ = 8,03 (1H,s); δ = 8,48 und 8,51 (1H,2s).

Beispiel 4

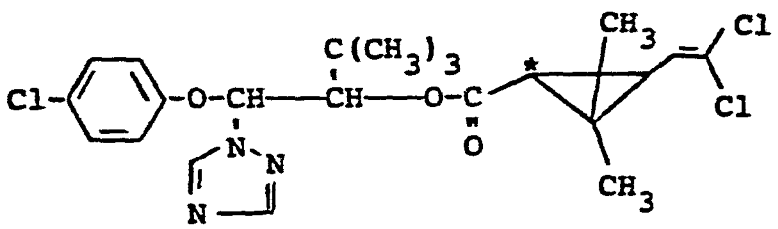

(I-4)

1S -trans

Diastereomeren-Gemisch
$H^1$-NMR-Spektrum (90 MHz):
δ = 0,98 - 1,27 (15H verschiedene s)
δ = 1,66 - 1,77 (1H,m); δ = 2,10 - 2,25 (1H,m)
δ = 5,15 - 5,25 (1H,m); δ = 5,60 - 5,65 (1H,m)
δ = 6,325 - 6,42 (1H,m); δ = 7,94 (1H,s)
δ = 6,70 - 7,30 (4H arom.); δ = 8,15 - 8,26 (1H,m).

0200146

Beispiel 5

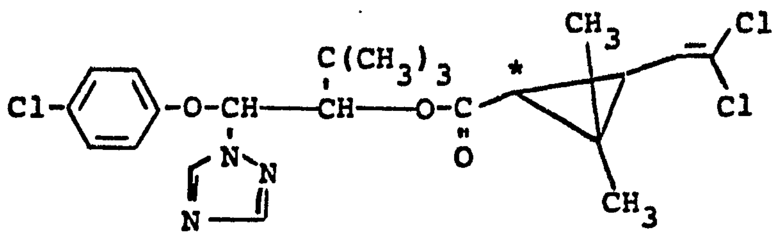

(I-5)

88 % threo-1R-trans Ester
12 % erythro-1R-trans Ester

$H^1$ - NMR Spektrum (90 MHz):
δ = 0,95 und 1,05 (9H,2S);
δ = 1,12, 1,20, 1,25 und 1,27 (6H,4s);
δ = 2,6 (1H,2d); δ = 2,1 - 2,36 (1H,m);
δ = 5,18 und 5,24 (1H,2d);
δ = 5,63 und 5,66 (1H,2d); δ = 6,32 und 6,38 (1H,2d);
δ = 6,7 - 7,4 (4H arom.);
δ = 7,95 und 7,98 (1H,2s);
δ = 8,22 und 8,25 (1H,2s).

Beispiel 6

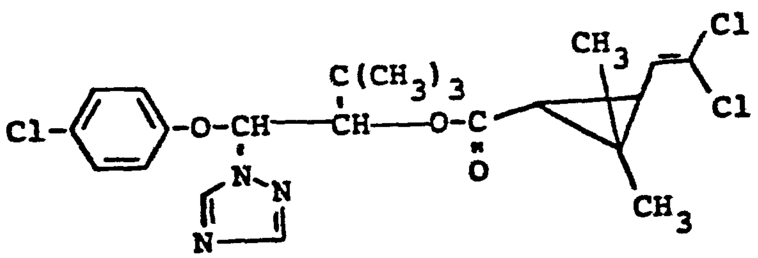

(I-6)

1S-trans Ester (4 Enantiomere)

Ein Diastereomeren-Gemisch von 1S-trans-Permethrinsäure-[1-(4-chlorphenoxy)-1-(1,2,4-triazol-1-yl)-3,3-dimethyl-but-2-yl]-estern (RR, SS, SR und RS - Enantiomere) wird getrennt, indem man eine 20 gew.-%ige Lösung des Diastereomeren-Gemisches in Chloroform chromatographiert.

Geräte: Lobarsystem bestehend aus
Pumpe: GFG Type 1 pr
Detektor: Knauer RI-Detektor
Autofraktionssammler: LKB 2111 Multirac

Eluierungsmittel: n-Hexan: tert.-Butyl-methyl-Ether = 6:4

Fließgeschwindigkeit: 6 ml pro Minute

Säule: Lichropräp. Si 60, Größe C

Aufgabemenge: 4 g
Retentionszeit: $t_R$ = 4-5,5 h

Die aufgefangenen Fraktionen mit identischem Inhalt werden vereinigt und eingedampft. Man erhält auf diese Weise jedes der vier Diastereomeren in einer Ausbeute von 70 % der Theorie. Die chemische Reinheit beträgt jeweils über 98 %; die optische Reinheit ist größer als 97 %.

Isomeres A:

$H^1$-NMR-Spektrum (90 MHz)
δ = 1,05 (9H,s); δ = 1,14 (3H,s);
δ = 1,21 (3H,s); δ = 1,70 (1H,d); δ = 2,17 (1H,d);
δ = 5,17 (1H,d); δ = 5,64 (1H,d); δ = 6,38 (1H,d);
δ = 6,78 (2H arom.); δ = 7,17 (2H arom.);
δ = 7,94 (1H,s); δ = 8,19 (1H,s).

Isomeres B:

$H^1$-NMR-Spektrum (90 MHz)
δ = 0,98 (9H,s); δ = 1,21 (3H,s); δ = 1,27 (3H,s);
δ = 1,66 (1H,d); δ = 2,23 (1H,dd);
δ = 5,21 (1H,d); δ = 5,61 (1H,d);
δ = 6,30 (1H,d); δ = 6,70 - 7,25 (4H arom.)
δ = 7,95 (1H,s); δ = 8,21 (1H,s):

2 Erythro-Enantiomere

Isomeres C:

$H^1$ - NMR-Spektrum (90 MHz)
δ = 0,95 (9H,s); δ = 1,19 (3H,s); δ = 1,24 (3H,s);
δ = 1,53 (1H,d); δ = 2,17 (1H,dd); δ = 5,39 (1H,d);
δ = 5,62 (1H,d); δ = 6,26 (1H,d);
δ = 6,7 - 7,2 (4H arom.);
δ = 7,90 (1H,s); δ = 8,34 (1H,s).

Isomeres D:

$H^1$-NMR-Spektrum (90MHz)
δ = 0,98 (9H,s); δ = 1,17 (6H,s); δ = 1,54 (1H,d);
δ = 2,19 (1H,dd); δ = 5,40 (1H,d); δ = 5,62 (1H,d);
δ = 6,26 (1H,d); δ = 6,70 - 7,28 (4H arom.)
δ = 7,93 (1H,s); δ = 8,32 (1H,s).

Beispiel A

Sphaerotheca-Test (Gurke) / protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit Konidien des Pilzes Sphaerotheca fuliginea bestäubt.

Die Pflanzen werden anschließend bei 23 bis 24°C und bei einer relativen Luftfeuchtigkeit von ca. 75 % im Gewächshaus aufgestellt.

10 Tage nach der Inokulation erfolgt die Auswertung.

In diesem Test zeigen die erfindungsgemäßen Verbindungen, die in den Beispielen 1, 2, 3, 4 und 5 offenbart sind, eine sehr gute Wirksamkeit.

## Patentansprüche

1. Permethrinsäure-(triazolyl-alkyl)-ester der Formel

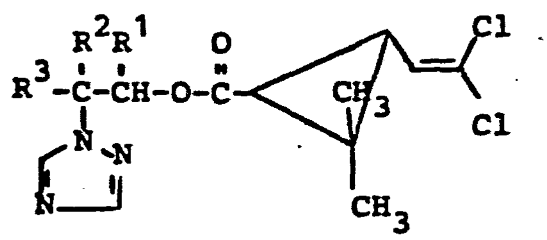

(I)

in welcher

$R^1$ für gegebenenfalls durch Halogen substituiertes tert.-Butyl steht,

$R^2$ für Wasserstoff steht und

$R^3$ für Alkyl, Alkoxy, Cycloalkylalkyl, gegebenenfalls substituiertes Aryl, gegebenenfalls substituiertes Aralkyl, gegebenenfalls substituiertes Aroxy oder gegebenenfalls substituiertes Aroxyalkyl steht oder

$R^2$ und $R^3$ gemeinsam für die Gruppe der Formel

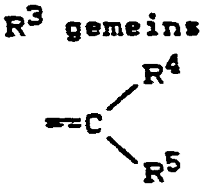

stehen, worin

$R^4$ für Wasserstoff, Alkyl, Cycloalkyl oder Cycloalkenyl steht und

$R^5$ für Alkyl, Cycloalkyl, Cycloalkenyl oder gegebenenfalls substituiertes Aryl steht oder

$R^4$ und $R^5$ gemeinsam mit dem Kohlenstoffatom, an das sie gebunden sind, für Cycloalkyl oder Cycloalkenyl stehen.

2. Permethrinsäure-(triazolyl-alkyl)-ester der Formel (I), in denen

$R^1$ für die Gruppierung

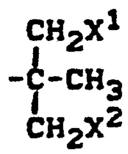

steht, worin

$X^1$ für Wasserstoff, Fluor oder Chlor steht und

$X^2$ für Wasserstoff, Fluor oder Chlor steht,

$R^2$ für Wasserstoff steht und

$R^3$ für Alkyl mit 1 bis 6 Kohlenstoffatomen, Alkoxy mit 1 bis 6 Kohlenstoffatomen, Cycloalkylalkyl mit 3 bis 8 Kohlenstoffatomen in der Cycloalkylgruppe und 1 bis 4 Kohlenstoffatomen im Alkylteil, gegebenenfalls ein- oder mehrfach, gleichartig oder verschieden durch Alkyl mit 1 bis 4 Kohlenstoffatomen, Halogen, Phenyl und/oder Nitro substituiertes

Aryl mit 6 bis 10 Kohlenstoffatomen, gegebenenfalls ein- oder mehrfach, gleichartig oder verschieden durch Alkyl mit 1 bis 4 Kohlenstoffatomen, Halogen, Phenyl und/oder Nitro substituiertes Aralkyl mit 6 bis 10 Kohlenstoffatomen im Arylteil und 1 bis 4 Kohlenstoffatomen im Alkylteil, für gegebenenfalls ein- oder mehrfach, gleichartig oder verschieden durch Alkyl mit 1 bis 4 Kohlenstoffatomen, Halogen, Phenyl und/oder Nitro substituiertes Aroxy mit 6 bis 10 Kohlenstoffatomen oder für gegebenenfalls ein- oder mehrfach, gleichartig oder verschieden durch Alkyl mit 1 bis 4 Kohlenstoffatomen, Halogen, Phenyl und/oder Nitro substituiertes Aroxyalkyl mit 6 bis 10 Kohlenstoffatomen im Aroxyteil und 1 bis 4 Kohlenstoffatomen im Alkylteil steht, und außerdem $R^2$ und $R^3$ gemeinsam für die Gruppe der Formel

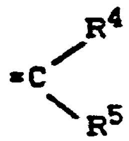

steht, worin

$R^4$ für Wasserstoff, Alkyl mit 1 bis 4 Kohlenstoffatomen, Cycloalkyl mit 3 bis 8 Kohlenstoffatomen oder Cycloalkenyl mit 5 bis 8 Kohlenstoffatomen steht und

$R^5$ für Alkyl mit 1 bis 4 Kohlenstoffatomen, Cycloalkyl mit 3 bis 8 Kohlenstoffatomen, Cycloalkenyl mit 5 bis 8 Kohlenstoffatomen oder für gegebenenfalls ein- oder

mehrfach, gleichartig oder verschieden durch Alkyl mit 1 bis 4 Kohlenstoffatomen und/oder Haloen substituiertes Aryl mit 6 bis 10 Kohlenstoffatomen steht, und außerdem

$R^4$ und $R^5$ auch gemeinsam mit dem Kohlenstoffatom, an das sie gebunden sind, für Cycloalkyl mit 5 bis 8 Kohlenstoffatomen oder für Cycloalkenyl mit 5 bis 8 Kohlenstoffatomen stehen.

3. Verfahren zur Herstellung von Permethrinsäure-(triazolyl-alkyl)estern der Formel

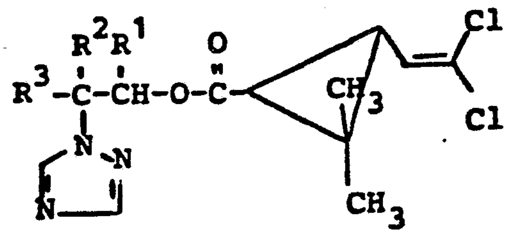

(I)

in welcher

$R^1$ für gegebenenfalls durch Halogen substituiertes tert.-Butyl steht,

$R^2$ für Wasserstoff steht und

$R^3$ für Alkyl, Alkoxy, Cycloalkylalkyl, gegebenenfalls substituiertes Aryl, gegebenenfalls substituiertes Aralkyl, gegebenenfalls substituiertes Aroxy oder gegebenenfalls substituiertes Aroxyalkyl steht oder

$R^2$ und $R^3$ gemeinsam für die Gruppe der Formel

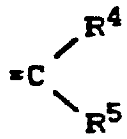 stehen, worin

$R^4$ für Wasserstoff, Alkyl, Cycloalkyl oder Cycloalkenyl steht und

$R^5$ für Alkyl, Cycloalkyl, Cycloalkenyl oder gegebenenfalls substituiertes Aryl steht oder

$R^4$ und $R^5$ gemeinsam mit dem Kohlenstoffatom, an das sie gebunden sind, für Cycloalkyl oder Cycloalkenyl stehen,

dadurch gekennzeichnet, daß man Azolderivate der Formel

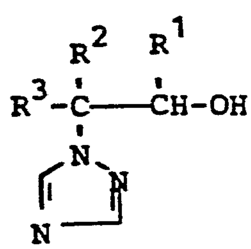

(II)

in welcher

$R^1$,$R^2$ und $R^3$ die oben angegebene Bedeutung haben,

mit Permethrinsäurehalogeniden der Formel

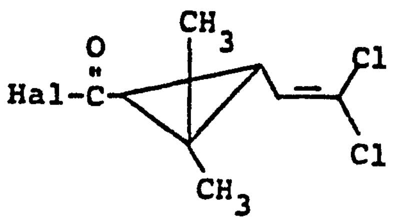

(III)

in welcher

Hal für Chlor oder Brom steht,

gegebenenfalls in Gegenwart eines Säurebindemittels und gegebenenfalls in Gegenwart eines Verdünnungsmittels umsetzt.

4. Fungizide Mittel, gekennzeichnet durch einen Gehalt an mindestens einem Permethrinsäure-(triazolyl-alkyl)-ester der Formel (I).

5. Verwendung von Permethrinsäure-(triazolyl-alkyl)-estern der Formel (I) zur Bekämpfung von Pilzen.

6. Verfahren zur Bekämpfung von Pilzen, dadurch gekennzeichnet, daß man Permethrinsäure-(triazolyl-alkyl)-ester der Formel (I) auf Pilze und/oder deren Lebensraum ausbringt.

7. Verfahren zur Herstellung von fungiziden Mitteln, dadurch gekennzeichnet, daß man Permethrinsäure-(triazolyl-alkyl)-ester der Formel (I) mit Streckmitteln und/oder oberflächenaktiven Stoffen vermischt.

8. Permethrinsäure-(triazolyl-alkyl)-ester gemäß Anspruch 1, gekennzeichnet durch die Formel

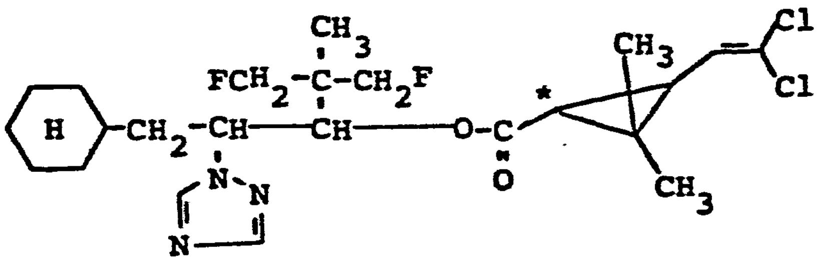

(I-1)

1R trans

9. Permethrinsäure-(triazolyl-alkyl)-ester gemäß Anspruch 1, gekennzeichnet durch die Formel

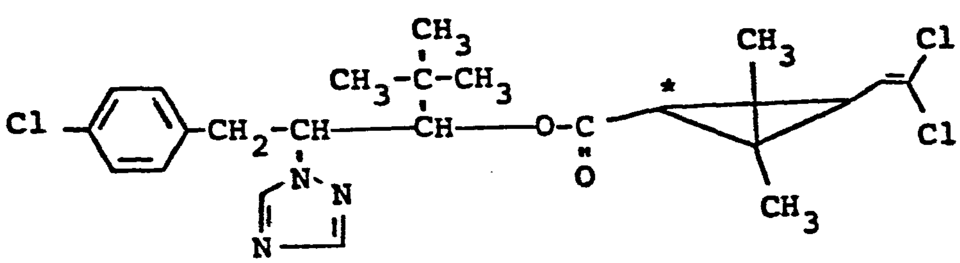

(I-2)

1 S-trans

10. Permethrinsäure-(triazolyl-alkyl)-ester gemäß Anspruch 1, gekennzeichnet durch die Formel

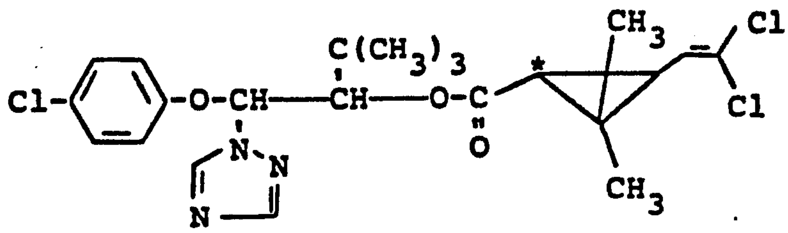

(I-4)

1S -trans